# FASCICULE DE BREVET EUROPEEN

(11) **EP 0 869 776 B1**
(45) Date de publication et mention de la délivrance du brevet: **06.05.2004**
(21) Numéro de dépôt: 96942419.1
(22) Date de dépôt: 18.12.1996
(51) Int. Cl.: A61K 9/51

(54) **NANOPARTICULES STABILISEES ET FILTRABLES DANS DES CONDITIONS STERILES**
STABILISIERTE UND STERIL FILTRIERBARE NANOPARTIKEL
STABILISED NANOPARTICLES CAPABLE OF BEING FILTERED UNDER STERILE CONDITIONS

(30) Priorité: 19.12.1995 FR 9515033
(43) Date de publication de la demande: 14.10.1998
(73) Titulaire: Aventis Pharma S.A., 92160 Antony (FR)
(72) Inventeur: VERRECCHIA, Thierry, F-94110 Arcueil (FR)
(86) Numéro de dépôt international: PCT/FR1996/002015
(87) Numéro de publication internationale: WO 1997/022337

(56) Documents cités:
- EP-A- 0 344 040
- EP-A- 0 520 888
- WO-A-91/15193
- US-A- 5 298 262

## Description

La présente invention concerne des nanoparticules de très petites dimensions, qui présentent, outre l'avantage de pouvoir circuler dans le flux sanguin sans problème de dimension au niveau des capillaires, les avantages d'être stabilisées, de pouvoir être filtrées de façon stérile, et de pouvoir être lyophilisées.

WO 91 15193 décrit des microsphères biodégradables et biocompatibles comprenant un polymère biodégradable et biocompatible et une substance tensioactive également biodégradable et biocompatible. Parmi les substances tensioactives mentionnées, pouvaient être utilisées des lécithines ou des sels biliaires, mais jamais de mélange lécithines/tensio-actifs.

Dans les demandes de brevet EP 523183, EP 520888 et EP 520889 ont été décrites des particules sphériques de petites dimensions présentant l'avantage d'être injectables. Cependant les nanoparticules ainsi préparées présentent des diamètres moyens de l'ordre de 50 à 500 nm et ne seraient pas stérilisables par filtration stérilisante sans une perte considérable de rendement, et/ou pas lyophilisables du fait d'une stabilité insuffisante.

Dans Eur. J. Pharm. Biopharm., 39(5), 173-191 (1993) les auteurs ont examiné les technologies actuellement disponibles dans le domaine des nanoparticules destinées à l'industrie pharmaceutique. Il est précisé page 182 que la filtration stérile de suspensions de nanoparticules n'a jamais été décrite.

Il a maintenant été trouvé, et c'est ce qui fait l'objet de la présente invention, que l'on peut préparer des particules dont le diamètre moyen est à 95 % inférieur à 100 nm, et plus précisément dont le diamètre moyen est compris entre 20 et 75 nm et qui peuvent donc être soumises à une filtration stérile sur des filtres de 0,22 µm sans perte du rendement. Ces particules sont en outre plus stables que celles qui pouvaient être obtenues selon l'art antérieur et peuvent être lyophilisées sans entraîner de phénomène d'agglomération des particules.

Selon l'invention, les nanoparticules comprennent au moins un polymère ou un copolymère hydrophobe, non hydrosoluble et non hydrodispersable, émulsionné dans une solution ou dispersion aqueuse de phospholipides et d'un sel de l'acide oléique, notamment l'oléate de sodium.

Selon l'invention un principe actif peut être introduit avec le polymère ou le copolymère dans les nanoparticules.

Les phospholipides sont choisis à titre d'exemple parmi les phospholipides naturels, synthétiques ou semi-synthétiques ; on utilise plus particulièrement les lécithines (phosphatidylcholine), comme par exemple les lécithines d'oeuf ou de soja purifiées (lécithine E100®, lécithine E80®, Phospholipons® par exemple Phospholipon 90®), la phosphatidyléthanolamine, la phosphatidylsérine, le phosphatidylinositol, le phosphatidylglycérol, la dipalmitoylphosphatidylcholine, la dipalmitoylglycérophosphatidylcholine, la dimyristoylphosphatidylcholine, la distéaroylphosphatidylcholine, l'acide phosphatidique ou leurs mélanges.

Le polymère ou le copolymère hydrophobe, non hydrosoluble et non hydrodispersable peut être choisi parmi les polymères biocompatibles et biodégradables, par exemple les polymères de l'acide lactique ou glycolique ainsi que leurs copolymères, ou les copolymères polylactique/polyoxyde d'éthylène (ou de propylène) de préférence de poids moléculaire compris entre 1000 et 200000, les polymères de l'acide polyhydroxybutyrique, les polylactones des acides gras contenant au moins 12 atomes de carbone ou les polyanhydrides.

Les nanoparticules selon l'invention sont tout à fait adaptées à une application à des principes actifs hydrophobes. Les principes actifs pouvant être utilisés peuvent être choisis parmi les grandes classes de médicaments destinés à la médecine humaine ou vétérinaire. Ils peuvent être également choisis parmi des principes destinés à l'industrie cosmétique, agro-alimentaire ou parmi les agents de diagnostic.

A titre d'exemple, des principes actifs intéressant l'industrie pharmaceutique peuvent être choisis, à titre non limitatif, parmi les antirhumatismaux, les anti-inflammatoires non stéroïdiens, les analgésiques, les anti-tussifs, les psychotropes, les stéroïdes, les barbituriques, les antimicrobiens, les anti-allergiques, les antiasthmatiques, les antispasmodiques et anti-sécrétoires, les cardiovasculaires et les vasodilatateurs cérébraux, les protecteurs cérébraux et les protecteurs hépatiques, les agents thérapeutiques du tractus gastrointestinal, les agents anti-cancéreux ou les agents anti-viraux, les vitamines, les contraceptifs, les vaccins ....

Selon l'invention, les nanoparticules peuvent être obtenues par la technique d'évaporation de solvant, à partir d'une solution ou d'une dispersion aqueuse de phospholipides et d'un sel de l'acide oléique dans laquelle est ajoutée une phase organique non miscible comprenant le principe actif et le polymère ou le copolymère hydrophobe, non hydrosoluble et non hydrodispersable. Le mélange est pré-émulsionné puis soumis à une homogénéisation et à l'évaporation du solvant organique pour obtenir une suspension aqueuse de nanoparticules de très petites dimensions.

La mise en oeuvre de ce procédé est décrite plus en détail dans les exemples.

La phase organique non miscible est de préférence choisie parmi les solvants volatils pouvant être de bons solvants du système polymère choisi. Par exemple on choisira des esters comme notamment l'acétate d'éthyle, des solvants chlorés, par exemple le dichlorométhane ou le chloroforme, ou encore parmi les cétones comme la méthyl éthyl cétone.

D'une manière générale le principe actif constitue de préférence environ 25 % en poids par rapport à la quantité de polymère introduite. Cependant cette quantité pourra varier, éventuellement être plus faible ou même aller jusqu'à 50 % en poids par rapport à la quantité de polymère ou de copolymère introduite.

La phase organique non miscible est constituée de telle manière que le principe actif et le polymère ou le copolymère représentent de 0,1 à 7 % en poids, de la solution.

La phase aqueuse constituée d'une solution ou d'une dispersion aqueuse de phospholipides et de sel de l'acide oléique comprend avantageusement ces constituants dans une proportion molaire respective de 1/1. Néanmoins cette proportion peut varier de telle manière que le rapport molaire phospholipides par rapport au sel de l'acide oléique soit de 0,1 à 1.

La phase aqueuse est constituée de telle manière que les phospholipides et le sel de l'acide oléique représentent au total de 0,1 à 2 % en poids dans la solution.

Les quantités relatives de phase organique et de phase aqueuse sont choisies de telle manière que la phase organique représente 20 à 60 % en volume par rapport à la phase aqueuse.

Les nanoparticules ainsi obtenues peuvent être filtrées sans donner lieu à des problèmes de colmatage et avec de bons rendements. La filtration s'effectue par filtrations en cascades sur filtres de porosités décroissantes, suivies d'une dernière filtration sur filtre de 0,22 µm.

De préférence, après la filtration, la suspension obtenue est lyophilisée en présence d'un ou plusieurs agents cryoprotecteurs. L'agent cryoprotecteur constitue 5 à 20 % (poids/volume) de la suspension soumise à la lyophilisation.

La solution destinée à la lyophilisation comprend certains additifs comme des composés non ioniques, par exemple un agent cryoprotecteur ou un agent destiné à ajuster l'isotonicité de la solution finale à injecter. Ces agents peuvent être choisis parmi des sucres (glucose, mannitol, sorbitol, saccharose par exemple), des polymères [par exemple dextran (dextran 1500, dextran 40000) polyvinylpyrrolidones injectables, polyéthylèneglycol ...], des acides aminés (par exemple le glycocolle), ou tout autre agent pouvant exercer cette fonction. Elle peut également contenir un/(des) agent(s) conservateur(s). Le cas échéant, le lyophilisat peut être repris au moment de l'emploi par de l'eau pour préparations injectables. Il est entendu que de telles opérations ne modifient pas la dimension des particules.

Les nanoparticules selon l'invention sont particulièrement intéressantes du fait de leur stabilité. Cette stabilité permet notamment d'obtenir un lyophilisat de bonne qualité dont la remise en solution et/ou en suspension, au cours de l'utilisation, est améliorée et pour lequel la suspension reconstituée contient des particules de diamètre voisin de celui des nanoparticules initiales.

Les nanoparticules selon l'invention peuvent être utilisées pour la préparation de compositions stériles destinées aux domaines pharmaceutiques ou vétérinaires, au domaine cosmétique, agro-alimentaire ou aux agents de diagnostics.

Cette technique est particulièrement intéressante du fait qu'elle ouvre la voie à la préparation à l'échelle industrielle de suspensions nanoparticulaires stabilisées et décontaminées, éventuellement chargées de principes actifs ce qui n'était pas possible jusqu'alors.

En outre les nanoparticules stabilisées selon l'invention présentent un avantage considérable dans le cas de certains principes actifs comme par exemple les agents anticancéreux de la classe des taxoïdes. Elles permettent en effet d'accroître l'activité du produit par comparaison à des formulations classiques comme notamment les formulations à base de mélange polysorbate/éthanol.

La présente invention concerne également les compositions pharmaceutiques constituées des nanoparticules selon l'invention, éventuellement en association avec un ou plusieurs excipients ou adjuvants compatibles et pharmaceutiquement acceptables. Ces compositions sont de préférence des compositions injectables.

L'administration par voie parentérale comprend les administrations intraveineuse, intrapéritonéale, intramusculaire ou sous-cutanée. Plus particulièrement préférée est l'administration intrapéritonéale ou intraveineuse.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Chez l'homme, les doses sont généralement comprises entre 0,01 et 200 mg/kg. Par voie intraveineuse, les doses sont généralement comprises entre 0,1 et 50 mg/kg, de préférence entre 0,1 et 8 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

Les exemples suivants, illustrent la présente invention.

### Exemple 1

300 mg (15 mg/ml théorique) d'un co-polymère dibloc constitué de l'association d'un poly(acide d,l-lactique) de masse 30 kD et d'un polyethylène glycol de masse 2 kD (PLA-PEG) sont dissous dans 8 ml d'acétate d'éthyle (solution A). 70 mg de lécithine E80 et 50 mg d'oléate de sodium sont dispersés dans 20 ml de solution glucosée à 5 % p/v (solution B). La solution A est émulsionnée dans la solution B par un Ultra-turrax puis la pré-émulsion est ensuite introduite dans un homogénéisateur de type Microfluidizer 110 S® pendant 3 minutes à 10°C. Le volume d'émulsion récupéré est d'environ 30 ml (30 g). L'acétate d'éthyle est éliminé à l'aide d'un évaporateur rotatif sous pression réduite (100 mm de mercure) jusqu'à un volume de suspension d'environ 17 ml (17 g). La suspension est filtrée sur deux filtres en série de porosité décroissante (1,2 µm Minisart NML® + 0,22 µm SLGS®).

Le diamètre moyen des particules mesuré par diffusion de lumière sur un appareil Brookhaven® est d'environ 44 nm.

La densité optique à 405 nm de la suspension avant et après la dernière filtration sur 0,22 µm est 0,44.

### Exemple 2

Une solution A est préparée de manière identique à l'exemple 1. 70 mg de lécithine E80 et 35 mg d'oléate de sodium sont dispersés dans 20 ml de solution glucosée à 5 % p/v (solution B). La solution A est émulsionnée dans la solution B par un Ultra-turrax puis la pré-émulsion est ensuite introduite dans un homogénéisateur de type Microfluidizer 110 S® pendant 3 minutes à 10°C. Le volume d'émulsion récupéré est d'environ 30 ml (30 g). L'acétate d'éthyle est éliminé à l'aide d'un évaporateur rotatif sous pression réduite (100 mm de mercure) jusqu'à un volume de suspension d'environ 17 ml (17 g). La suspension est filtrée sur deux filtres en série de porosité décroissante (1,2 µm Minisart NML® + 0,22 µm SLGS®).

Le diamètre moyen des particules mesuré par diffusion de lumière sur un appareil Brookhaven® est d'environ 46 nm.

La densité optique à 405 nm de la suspension avant et après la dernière filtration sur 0,22 µm est 0,64.

### Exemple 3

Une solution A est préparée de manière identique à l'exemple 1. 70 mg de lécithine E80 et 20 mg d'oléate de sodium sont dispersés dans 20 ml de solution glucosée à 5 % p/v (solution B). La solution A est émulsionnée dans la solution B par un Ultra-turrax puis la pré-émulsion est ensuite introduite dans un homogénéisateur de type Microfluidizer 110 S® pendant 3 minutes à 10°C. Le volume d'émulsion récupéré est d'environ 30 ml (30 g). L'acétate d'éthyle est éliminé à l'aide d'un évaporateur rotatif sous pression réduite (100 mm de mercure) jusqu'à un volume de suspension d'environ 17 ml (17 g). La suspension est filtrée sur deux filtres en série de porosité décroissante (1,2 µm Minisart NML® + 0,22 µm SLGS®).

Le diamètre moyen des particules mesuré par diffusion de lumière sur un appareil Brookhaven® est d'environ 58 nm.

La densité optique à 405 nm de la suspension avant et après la dernière filtration sur 0,22 µm est 1.20.

### Exemple 4

Une solution A est préparée de manière identique à l'exemple 1. 70 mg de lécithine E80 et 20 mg d'oléate de sodium sont dispersés dans 20 ml de solution glucosée à 10 % p/v (solution B). La solution A est émulsionnée dans la solution B par un Ultra-turrax puis la pré-émulsion est ensuite introduite dans un homogénéisateur de type Microfluidizer 110 S® pendant 3 minutes à 10°C. Le volume d'émulsion récupéré est d'environ 30 ml (30 g). L'acétate d'éthyle est éliminé à l'aide d'un évaporateur rotatif sous pression réduite (100 mm de mercure) jusqu'à un volume de suspension d'environ 17 ml (17 g). La suspension est filtrée sur deux filtres en série de porosité décroissante (1,2 µm Minisart NML® + 0,22 µm SLGS®).

Le diamètre moyen des particules mesuré par diffusion de lumière sur un appareil Brookhaven® est d'environ 61 nm.

La densité optique à 405 nm de la suspension avant et après la dernière filtration sur 0,22 µm est 0,90.

### Exemple 5

Une solution A est préparée de manière identique à l'exemple 1. 70 mg de lécithine E80 et 20 mg d'oléate de sodium sont dispersés dans 20 ml d'une solution de maltose à 5 % p/v (solution B). La solution A est émulsionnée dans la solution B par un Ultra-turrax puis la pré-émulsion est ensuite introduite dans un homogénéisateur de type Microfluidizer 110 S® pendant 3 minutes à 10°C. Le volume d'émulsion récupéré est d'environ 30 ml (30 g). L'acétate d'éthyle est éliminé à l'aide d'un évaporateur rotatif sous pression réduite (100 mm de mercure) jusqu'à un volume de suspension d'environ 17 ml (17 g). La suspension est filtrée sur deux filtres en série de porosité décroissante (1,2 µm Minisart NML® + 0,22 µm SLGS®).

Le diamètre moyen des particules mesuré par diffusion de lumière sur un appareil Brookhaven® est d'environ 57 nm.

La densité optique à 405 nm de la suspension avant et après la dernière filtration sur 0,22 µm est 1,10.

### Exemple 6

750 mg (15 mg/ml théorique) d'un co-polymère dibloc constitué de l'association d'un poly(acide d,l-lactique) de masse 30 kD et d'un polyethylène glycol de masse 2 kD (PLA-PEG) et 250 mg (5 mg/ml théorique) de tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α sont dissous dans 20 ml d'acétate d'éthyle (solution A). 175 mg de lécithine E80 et 90 mg d'oléate de sodium sont dispersés dans 50 ml de solution glucosée à 5 % p/v (solution B). La solution A est émulsionnée dans la solution B par un Ultra-turrax puis la pré-émulsion est ensuite introduite dans un homogénéisateur de type Microfluidizer 110 S® pendant 10 minutes à 10°C. Le volume d'émulsion récupéré est d'environ 70 ml (70 g). L'acétate d'éthyle est éliminé à l'aide d'un évaporateur rotatif sous pression réduite (100 mm de mercure) jusqu'à un volume de suspension d'environ 45 ml (45 g). La suspension est filtrée sur deux filtres en série de porosité décroissante (1,2 µm Minisart NML® + 0,22 µm SLGS®). La suspension filtrée est stérile.

Le diamètre moyen des particules mesuré par diffusion de lumière sur un appareil Brookhaven® est d'environ 66 nm.

La densité optique à 405 nm de la suspension avant et après la dernière filtration sur 0,22 µm est 2,8.

Le rendement de fabrication, exprimé par le rapport de la concentration en tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α finale après filtration, sur la concentration théorique initiale (5 mg/ml), est supérieure à 90 %.

La concentration en PLA-PEG (calculée par rapport au rendement en tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α est 14 mg/ml.

La suspension ne subit aucune modification chimique (absence de dégradation de la matière active) et physique (la taille des particules et la densité optique restent identiques) après 4 mois de conservation à 4°C et 25°C.

Le tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α peut être préparé comme décrit dans la demande de brevet WO 94/13654.

### Exemple 7

750 mg (15 mg/ml théorique) d'un co-polymère dibloc constitué de l'association d'un poly(acide d,l-lactique) de masse 30 kD et d'un polyethylène glycol de masse 2 kD (PLA-PEG) sont dissous dans 20 ml d'acétate d'éthyle (solution A). 175 mg de lécithine E80 et 90 mg d'oléate de sodium sont dispersés dans 50 ml de solution glucosée à 5 % p/v (solution B). La solution A est émulsionnée dans la solution B par un Ultra-turrax puis la pré-émulsion est ensuite introduite dans un homogénéisateur de type Microfluidizer 110 S® pendant 10 minutes à 10°C. Le volume d'émulsion récupéré est d'environ 70 ml (70 g). L'acétate d'éthyle est éliminé à l'aide d'un évaporateur rotatif sous pression réduite (100 mm de mercure) jusqu'à un volume de suspension d'environ 45 ml (45 g). La suspension est filtrée sur deux filtres en série de porosité décroissante (1,2 µm Minisart NML® + 0,22 µm SLGS®). La suspension filtrée est stérile.

Le diamètre moyen des particules mesuré par diffusion de lumière sur un appareil Brookhaven® est d'environ 63 nm.

La densité optique à 405 nm de la suspension avant et après la dernière filtration sur 0,22 µm est 1,6.

La suspension ne subit aucune modification physique (la taille des particules et la densité optique restent identiques) après 4 mois de conservation à 4°C et 25°C.

### Exemple 8

750 mg (15 mg/ml théorique) d'un co-polymère dibloc constitué de l'association d'un poly(acide d,l-lactique) de masse 30 kD et d'un polyethylène glycol de masse 2 kD (PLA-PEG) et 250 mg (5 mg/ml théorique) de tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α sont dissous dans 10 ml d'acétate d'éthyle (solution A). 175 mg de lécithine E80 et 45 mg d'oléate de sodium sont dispersés dans 50 ml de solution glucosée à 5 % p/v (solution B). La solution A est émulsionnée dans la solution B par un Ultra-turrax puis la pré-émulsion est ensuite introduite dans un homogénéisateur de type Microfluidizer 110 S® pendant 10 minutes à 10°C. Le volume d'émulsion récupéré est d'environ 60 ml (60 g). L'acétate d'éthyle est éliminé à l'aide d'un évaporateur rotatif sous pression réduite (100 mm de mercure) jusqu'à un volume de suspension d'environ 36 ml (36 g). La suspension est filtrée sur deux filtres en série de porosité décroissante (1,2 µm Minisart NML® + 0, 22 µm SLGS®). La suspension filtrée est stérile.

Le diamètre moyen des particules mesuré par diffusion de lumière sur un appareil Brookhaven® est d'environ 64 nm.

La densité optique à 405 nm de la suspension avant et après la dernière filtration sur 0,22 µm est 1,5.

Le rendement de fabrication, exprimé par le rapport de la concentration en tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α finale après filtration, sur la concentration théorique initiale (5 mg/ml), est supérieure à 90 %.

La proportion de tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α encapsulée dans les nanoparticules, mesuré par dosage du surnageant après ultracentrifugation (65000 G, 2 h), est de l'ordre de 98 %.

La concentration en PLA-PEG (calculée par rapport au rendement en tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α est 14 mg/ml.

Le tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α peut être préparé comme décrit dans la demande de brevet WO 94/13654.

### Exemple 9

3.0 g (15 mg/ml théorique) d'un co-polymère dibloc constitué de l'association d'un poly(acide d,l-lactique) de masse 30 kD et d'un polyethylène glycol de masse 2 kD (PLA-PEG) sont dissous dans 80 ml d'acétate d'éthyle (solution A). 700 mg de lécithine E80 et 180 mg d'oléate de sodium sont dispersés dans 200 ml d'eau pour préparation injectable (solution B). La solution A est émulsionnée dans la solution B par un Ultra-turrax puis la pré-émulsion est ensuite introduite dans un homogénéisateur de type Microfluidizer 110 S® pendant 10 minutes à 10°C. Le volume d'émulsion récupéré est d'environ 230 ml (230 g). L'acétate d'éthyle est éliminé à l'aide d'un évaporateur rotatif sous pression réduite (100 mm de mercure) jusqu'à un volume de suspension d'environ 186 ml (186 g). Le volume de la suspension est ajusté à un volume de 200 ml par de l'eau pour préparation injectable. La suspension est filtrée sur deux filtres en série de porosité décroissante (1,2 µm Minisart NML® + 0,22 µm SLGS®). La suspension filtrée, stérile, est lyophilisée en présence de 20 % p/v de saccharose.

Le diamètre moyen des particules mesuré par diffusion de lumière sur un appareil Brookhaven® avant la lyophilisation et après reprise du lyophilisat par le même volume d'eau pour préparation injectable, est compris entre 70 et 100 nm.

## Revendications

1. Suspension aqueuse de nanoparticules stabilisées et filtrables dans des conditions stériles **caractérisée en ce que** les nanoparticules comprennent au moins un polymère ou un copolymère hydrophobe, non hydrosoluble et non hydrodispersable, émulsionné dans une solution ou dispersion aqueuse de phospholipides et d'un sel de l'acide oléique.

2. Suspension aqueuse de nanoparticules stabilisées et filtrables dans des conditions stériles, selon la revendication 1, **caractérisée en ce qu'**un principe actif est introduit avec le polymère ou le copolymère.

3. Suspension aqueuse de nanoparticules stabilisées et filtrables dans des conditions stériles, selon l'une des revendications 1 ou 2, **caractérisée en ce que** le sel de l'acide oléique est l'oléate de sodium.

4. Suspension aqueuse de nanoparticules stabilisées et filtrables dans des conditions stériles, selon l'une des revendications 1 à 3, **caractérisée en ce que** le polymère ou le copolymère hydrophobe, non hydrosoluble et non hydrodispersable est choisi parmi les polymères biocompatibles et biodégradables

5. Procédé de préparation d'une suspension aqueuse de nanoparticules selon l'une des revendications 1 à 4, **caractérisé en ce que** l'on prépare une solution ou une dispersion aqueuse de phospholipides et d'un sel de l'acide oléique dans laquelle est ajoutée une phase organique non miscible comprenant le polymère ou le copolymère hydrophobe, non hydrosoluble et non hydrodispersable et le cas échéant le principe actif, puis on effectue une pré-émulsion et soumet le mélange à une homogénéisation et à l'évaporation du solvant organique, puis éventuellement filtre la suspension obtenue et éventuellement lyophilise.

6. Utilisation d'une suspension aqueuse de nanoparticules selon l'une des revendications 1 à 4 pour la préparation de compositions stériles après filtration stérilisante.

7. Utilisation selon la revendication 6, **caractérisé en ce que** la filtration stérilisante est effectuée en cascades, sur filtres de porosités décroissantes.

8. Suspension aqueuse de nanoparticules, selon l'une des revendications 1 à 4, **caractérisée en ce que** qu'elle est lyophilisée.

9. Suspension aqueuse de nanoparticules, selon l'une des revendications 1 à 4, **caractérisée en ce que** qu'elle est soumise à des opérations de filtration stérilisante, de lyophilisation et de remise en solution.

10. Composition pharmaceutique constituée d'une suspension aqueuse nanoparticules selon l'une des revendications 1 à 4 ou 8 ou 9, éventuellement en association avec un ou plusieurs excipients ou adjuvants compatibles et pharmaceutiquement acceptables.

## Claims

1. Aqueous suspension of stabilized nanoparticles which are filterable under sterile conditions, **characterized in that** the nanoparticles comprise at least one hydrophobic, water-insoluble and water-indispersible polymer or copolymer emulsified in an aqueous dispersion or solution of phospholipids and of an oleic acid salt.

2. Aqueous suspension of stabilized nanoparticles which are filterable under sterile conditions, according to Claim 1, **characterized in that** an active principle is introduced with the polymer or the copolymer.

3. Aqueous suspension of stabilized nanoparticles which are filterable under sterile conditions, according to either of Claims 1 and 2, **characterized in that** the oleic acid salt is sodium oleate.

4. Aqueous suspension of stabilized nanoparticles which are filterable under sterile conditions, according to one of Claims 1 to 3, **characterized in that** the hydrophobic, water-insoluble and water-indispersible polymer or copolymer is chosen from biocompatible and biodegradable polymers.

5. Process for the preparation of an aqueous suspension of nanoparticles according to one of Claims 1 to 4, **characterized in that** an aqueous dispersion or solution of phospholipids and of an oleic acid salt is prepared, into which is added an immiscible organic phase comprising the hydrophobic, water-insoluble and water-indispersible polymer or copolymer and, where appropriate, the active principle, after which a pre-emulsion is prepared and the mixture is subjected to homogenization and evaporation of the organic solvent, then the suspension obtained is optionally filtered and optionally lyophilized.

6. Use of an aqueous suspension of nanoparticles according to one of Claims 1 to 4 for the preparation of compositions which are sterile after sterilizing filtration.

7. Use according to Claim 6, **characterized in that** the sterilizing filtration is carried out in cascades, on filters of decreasing porosity.

8. Aqueous suspension of nanoparticles, according to one of Claims 1 to 4, **characterized in that** it is lyophilized.

9. Aqueous suspension of nanoparticles, according to one of Claims 1 to 4, **characterized in that** it undergoes sterilizing filtration, lyophilization and redissolution operations.

10. Pharmaceutical composition consisting of an aqueous suspension of nanoparticles according to one of Claims 1 to 4 or 8 or 9, optionally in combination with one or more compatible and pharmaceutically acceptable excipients or adjuvants.

## Patentansprüche

1. Wäßrige Suspension von Nanopartikeln, stabilisiert und filtrierbar unter sterilen Bedingungen, **dadurch gekennzeichnet, daß** die Nanopartikel mindestens ein hydrophobes, nicht wasserlösliches und nicht wasserdispergierbares Polymer oder Copolymer umfassen, emulgiert in einer wäßrigen Lösung oder Dispersion von Phospholipiden und einem Salz der Oleinsäure.

2. Wäßrige Suspension von Nanopartikeln, stabilisiert und filtrierbar unter sterilen Bedingungen, nach Anspruch 1, **dadurch gekennzeichnet, daß** zusammen mit dem Polymer oder dem Copolymer ein Wirkstoff eingebracht wird.

3. Wäßrige Suspension von Nanopartikeln, stabilisiert und filtrierbar unter sterilen Bedingungen, nach einem der Ansprüche 1 oder 2, **dadurch gekennzeichnet, daß** das Salz der Oleinsäure Natriumoleat ist.

4. Wäßrige Suspension von Nanopartikeln, stabilisiert und filtrierbar unter sterilen Bedingungen, nach einem der Ansprüche 1 bis 3, **dadurch gekennzeichnet, daß** das hydrophobe, nicht wasserlösliche und nicht wasserdispergierbare Polymer oder Copolymer unter den biokompatiblen und bioabbaubaren Polymeren ausgewählt wird.

5. Verfahren zur Herstellung einer wäßrigen Suspension von Nanopartikeln nach einem der Ansprüche 1 bis 4, **dadurch gekennzeichnet, daß** man eine wäßrige Lösung oder Dispersion von Phospholipiden und einem Salz der Oleinsäure herstellt, zu der man eine nicht mischbare organische Phase gibt, die das hydrophobe, nicht wasserlösliche und nicht wasserdispergierbare Polymer oder Copolymer und gegebenenfalls den Wirkstoff umfaßt, und man anschließend ein Pre-Emulgieren durchführt und die Mischung einer Homogenisierung und einem Verdampfen des organischen Lösungsmittels unterzieht, wonach man die erhaltene Suspension gegebenenfalls filtriert und gegebenenfalls lyophilisiert.

6. Verwendung einer wäßrigen Suspension von Nanopartikeln nach einem der Ansprüche 1 bis 4 zur Herstellung von sterilen Zusammensetzungen nach der sterilisierenden Filtration.

7. Verwendung nach Anspruch 6, **dadurch gekennzeichnet, daß** die sterilisierende Filtration in Kaskaden auf Filtern mit abnehmender Porosität durchgeführt wird.

8. Wäßrige Suspension von Nanopartikeln nach einem der Ansprüche 1 bis 4, **dadurch gekennzeichnet, daß** sie lyophilisiert ist.

9. Wäßrige Suspension von Nanopartikeln nach einem der Ansprüche 1 bis 4, **dadurch gekennzeichnet, daß** sie Operationen zur sterilisierenden Filtration, zur Lyophilisierung und zur Wiederauflösung unterzogen wird.

10. Pharmazeutische Zusammensetzung, bestehend aus einer wäßrigen Suspension von Nanopartikeln nach einem der Ansprüche 1 bis 4 oder 8 oder 9, gegebenenfalls in Assoziation mit einem oder mehreren kompatiblen und pharmazeutisch akzeptablen Füllstoffen oder Zusatzstoffen.
